# EUROPEAN PATENT APPLICATION

(11) **EP 0 722 945 A1**
(43) Date of publication of application: **24.07.1996**
(21) Application number: 96300338.9
(22) Date of filing: 17.01.1996
(51) Int. Cl.: C07D 487/04, C07F 9/568, A61K 31/55

(54) **9-oxo-1-azabicyclo(5.2.0)non-2,3-diene-2-carboxylic acid derivatives as antibiotics**

(30) Priority: 18.01.1995 EP 95400094
(71) Applicant: ZENECA LIMITED, London W1Y 6LN (GB); ZENECA Pharma S.A., F-95022 Cergy Pontoise Cédex (FR)
(72) Inventor: Koza, Patrice, Zeneca Pharma S.A., "Le Galien", F-95022 Cergy Cedex (FR); Lohmann, Jean-Jacques, Zeneca Pharma S.A., F-95022 Cergy Cedex (FR)
(74) Representative: Tinsley, Rachel Maria

(57) **Abstract**

The present invention provides a compound of the formula (I): 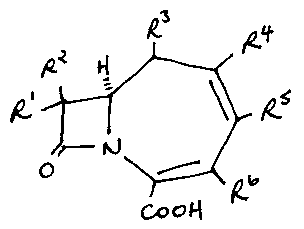
wherein R¹ is hydrogen, optionally substituted: acylamino, C₁₋₆alkyl, C₂₋₆alkenyl, C₂₋₆alkynyl, C₁₋₆alkoxy, C₁₋₆alkanoyloxy, C₁₋₆alkylamino, di-(C₁₋₆alkyl)amino, C₁₋₆alkoxycarbonyl, aminocarbonyl, C₁₋₆alkylaminocarbonyl or di-(C₁₋₆alkyl)aminocarbonyl;
R² is hydrogen or C₁₋₆alkoxy;
or R¹ and R² together form optionally substituted C₁₋₆alkylene;
R³ and R⁵ are independently hydrogen or C₁₋₆alkyl; and
one of R⁴ and R⁶ is hydrogen or C₁₋₄alkyl and the other is selected from a number of groups or a pharmaceutically acceptable salt or in vivo hydrolysable ester thereof.

Processes for their preparation, intermediates in their preparation, their use as therapeutic agents and pharmaceutical compositions containing them.

## Description

The invention relates to 9-oxo-1-azabicyclo[5.2.0]non-2,4- diene-2-carboxylic acid compounds, to processes for their preparation, to intermediates in their preparation, to their use as therapeutic agents and to pharmaceutical compositions containing them.

The compounds of this invention are β-lactam antibiotics and can be used in the treatment of any disease that is conventionally treated with antibiotics for example in the treatment of bacterial infection in mammals including humans.

Known classes of β-lactam antibacterial compound include cephalosporins, carbacephems, oxacephems, carbapenems and penems. Prior to this discovery, the ring systems of the present invention were not known to possess antibacterial properties.

The compounds referred to herein are named in accordance with the following nomenclature:

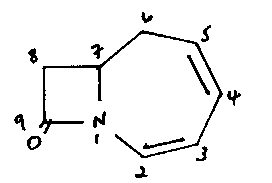

Accordingly the present invention provides a compound of the formula (I): 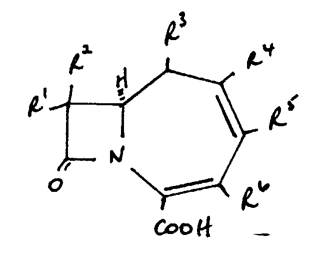 wherein
R¹ is hydrogen, optionally substituted: acylamino, C₁₋₆alkyl, C₂₋₆alkenyl, C₂₋₆alkynyl, C₁₋₆alkoxy, C₁₋₆alkanoyloxy, C₁₋₆alkylamino, di-(C₁₋₆alkyl)amino, C₁₋₆alkoxycarbonyl, aminocarbonyl, C₁₋₆alkylaminocarbonyl or di-(C₁₋₆alkyl)aminocarbonyl;
R² is hydrogen or C₁₋₆alkoxy;
or R¹ and R² together form optionally substituted C₁₋₆alkylene;
R³ and R⁵ are independently hydrogen or C₁₋₆alkyl; and
one of R⁴ and R⁶ is hydrogen or C₁₋₄alkyl and the other is selected from hydrogen, cyano, C₁₋₄alkylcarbonyl, C₁₋₄alkoxycarbonyl, benzoyl, C₁₋₄alkylsulphonyl, benzylsulphonyl, nitro, chloro, bromo, optionally substituted C₁₋₁₀alkyl, optionally substituted aryl, a group of the formula -SR⁷; wherein R⁷ is optionally substituted: C₁₋₁₀alkyl, C₂₋₆alkenyl, C₂₋₆alkynyl, C₃₋₁₀cycloalkyl, C₃₋₁₀cycloalkylC₁₋₃alkyl, aryl, arylC₁₋₃alkyl, heterocyclyl, heterocyclylC₁₋₃alkyl, heteroaryl or heteroarylC₁₋₃alkyl;
or one of R⁴ and R⁶ is hydrogen or C₁₋₄alkyl and the other is of the formula -OR¹⁸, wherein R¹⁸ is hydrogen, optionally substituted:
C₁₋₆alkyl, aryl, C₁₋₆alkanoyl or arylcarbonyl;
or one of R⁴ and R⁶ is hydrogen or C₁₋₄alkyl and the other is of the formula -N(R¹⁹)R²⁰ wherein R¹⁹ and R²⁰ are independently hydrogen, optionally substituted C₁₋₆alkyl, C₁₋₆alkanoyl, arylC₁₋₃alkanoyl, arylcarbonyl, heterocyclyl, heterocydylC₁₋₃alkyl, heteroaryl, heteroarylC₁₋₃alkyl, arylC₁₋₃alkyl, cycloalkyl or cycloalkylC₁₋₃alkyl;
or one of R⁴ and R⁶ is hydrogen or C₁₋₄alkyl and the other is of the formula -CH₂R²¹ wherein R²¹ is aryl or of the formula -SR⁷, -OR¹⁸ or -N(R¹⁹)R²⁰ wherein R⁷ and R¹⁸-R²⁰ are as hereinabove defined;
or a pharmaceutically acceptable salt or in vivo hydrolysable ester thereof.

Suitable substituents for carbon atoms in optionally substituted R¹ and R² groups include, halo, hydroxy, amino, C₁₋₄alkoxy, cyano, nitro, C₁₋₄alkyl, C₁₋₄alkylthio, C₁₋₄alkanoyl, carboxy, C₁₋₄alkoxycarbonyl, C₁₋₄alkylsulphinyl, C₁₋₄alkylsulphonyl, triazolyl and tetrazolyl.

Suitable substituents for optionally substituted R⁴ and R⁶ groups include hydroxy, halo, C₁₋₄alkoxy, cyano, amino, C₁₋₆alkylamino, di-(C₁₋₆alkyl)amino, C₁₋₆alkanoylamino, C₁₋₄alkoxycarbonyl, carboxy, iminoC₁₋₆alkyl, C_{1 -6}alkyliminoC₁₋₄alkyl, iminomethyl, C₁₋₆alkylimino, C₁₋₆alkylthio, C₁₋₆alkylsulphinyl, C₁₋₆alkylsulphonyl, arylC₁₋₄alkanoyl, aryloxyalkanoyl, oxo, a group of the formula -N=C(R⁸)R⁹, -N(R¹⁰)C(R¹¹)=NR¹², -C(N(R¹³)R¹⁴)=NR¹⁵, -CON(R¹⁶)R¹⁷ wherein R⁸ is amino, C₁₋₄alkylamino or di-(C₁₋₄alkyl)amino;
R⁹⁻R¹⁵ are independently hydrogen or C₁₋₄alkyl;
R¹⁶ is hydrogen or C₁₋₄alkyl;
R¹⁷ is hydrogen, C₁₋₄alkyl, aryl, heteroaryl, heterocyclyl, cycloalkyl or cycloalkylC₁₋₃alkyl.

Preferably R¹ is alkyl optionally substituted by halo, hydroxy, C₁₋₆alkylthio, C₁₋₆dkanoyl, C₁₋₆alkylsulphinyl, C₁₋₆alkylsulphonyl, triazolyl, tetrazolyl or acylamino. Most preferably R¹ is C₁₋₄alkyl optionally substituted by halo or hydroxy or of the formula R⁵⁰⁻C(=N-OR⁵¹)-, wherein R⁵⁰ and R⁵¹ are as hereinbelow defined.

In particular R¹ is 1-hydroxyethyl, 1-fluoroethyl or hydroxymethyl.

In another aspect R¹ is 2-(2-aminothiazol-4-yl)-2-(1-carboxy-1-methylethoxyimino)acetamido.

Preferably R² is hydrogen.

Preferably R³ is hydrogen or methyl.

Preferably R⁵ is hydrogen.

Preferably one of R⁴ and R⁶ is hydrogen and the other is hydrogen, optionally substituted C₁₋₁₀alkyl, optionally substituted aryl, a group of the formula -S(O)ₙR⁷ wherein n is 0, 1 or 2 and R⁷ is optionally substituted: C₁₋₆alkyl or heterocyclyl wherein optional substituents on C₁₋₆alkyl are selected from amino, C₁₋₄alkylamino, di-(C₁₋₄alkyl)amino or of the formula: -N=C(R⁸)R⁹, -N(R¹⁰C(R¹¹)=N-R¹² and -C(N(R¹³)R¹⁴)=N-R¹⁵ wherein R⁸⁻R¹⁵ are as hereinabove defined and optional substituents on heterocyclyl groups are selected from C₁₋₄alkyl, carboxy, C₁₋₄alkoxy, carbonyl, -CON(R¹⁶)R¹⁷ wherein R¹⁶ and R¹⁷ are as hereinabove defined; of the formula -OR¹⁸ wherein R¹⁸ is as hereinabove defined; of the formula -N(R¹⁹)R²⁰ wherein R¹⁹ and R²⁰ are independently selected from hydrogen, C₁₋₄alkyl, C₁₋₄alkanoyl and aryl;
or of the formula -CH₂R²¹ wherein R²¹ is as hereinabove defined.

In particular, one of R⁴ and R⁶ is of the formula -SR⁷.

In particular, R⁷ is of the formula 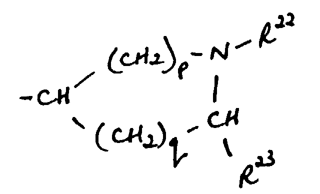 wherein p is 1 or 2, q is 1 or 2, R²² is hydrogen or C₁₋₄alkyl and R²³ is hydrogen, carboxy, C₁₋₄alkoxycarbonyl, -CON(R¹⁶)R¹⁷ wherein R¹⁶ and R¹⁷ are independently hydrogen, C₁₋₄alkyl, phenyl or thienyl. In particular, when R¹⁶ or R¹⁷ is phenyl or thienyl, the phenyl or thienyl group is substituted by at least one carboxy substituent.

In another aspect, one of R⁴ and R⁶ is of the formula -S(O)ₙR⁷ wherein n is 0 or 2 and R⁷ is optionally substituted C₁₋₆alkyl.

Most preferably one of R⁴ and R⁶ is C₁₋₄alkanoyloxy or of the formula -S(O)ₙR⁷ wherein n is 0 or 2 and R⁷ is C₁₋₆alkyl optionally substituted by amino or one of R⁴ and R⁶ is of the formula -SR⁷ wherein R⁷ is of the formula: 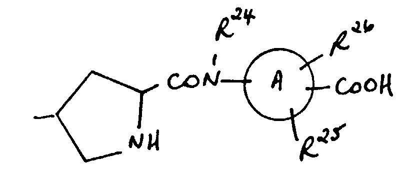 wherein R²⁴ is hydrogen or C₁₋₄alkyl and A is a phenyl or thienyl ring; and R²⁵ and R²⁶ are the same or different and are selected from hydrogen, halo, cyano, C₁₋₄alkyl, nitro, hydroxy, carboxy, C₁₋₄alkoxy, C₁₋₄alkoxycarbonyl, aminosulphonyl, C₁₋₄alkylaminosusphonony di-C₁₋₄₋alkylaminosulphonyl, carbamoyl, C₁₋₄alkylcarbamoyl, di-C₁₋₄alkylcarbamoyl, trifluoromethyl, sulphonic acid, amino, C₁₋₄alkylamino, di-C₁₋₄alkylamino, C₁₋₄alkanoylamino, C₁₋₄alkanoyl(N-C₁₋₄alkyl)amino, C₁₋₄alkanesulphonamido and C₁₋₄alkylS(O)ₙ₋ wherein n is zero, one or two.

'Alkyl' when used herein includes straight chain and branched chain substituents for example methyl, ethyl, n-propyl, isopropyl, n-butyl and isobutyl.

'Aryl' is used herein to describe optionally substituted, phenyl or naphthyl.

'Heteroaryl' is used herein to describe optionally substituted, preferably 5 or 6-membered mono- or 8-10 member bi-aromatic, groups having 1-4 ring heteroatoms, selected from nitrogen, oxygen and sulphur. For example, pyridyl, pyrimidyl, pyrazinyl, pyridazinyl, thienyl, thiazolyl, oxazolyl, thiadiazolyl, imidazyl, pyrrolyl, isothiazolyl, quinolyl, isoquinolyl, quinoxalinyl, quinoxalinyl and cinnolinyl.

'C₃₋₁₀cycloalkyl' is used herein to describe optionally substituted, saturated carbocyclic ring systems. For example cyclopropyl, cyclobutyl, cyclopentyl and cyclohexyl.

'Heterocyclyl' is used herein to describe optionally substituted, saturated or partially saturated 5-6 membered mono or 8-10 membered bicyclic ring systems having 1-4 ring heteroatoms selected from nitrogen, oxygen and sulphur. For example piperidyl, pyrrolidinyl, piperazinyl, morpholinyl, pyrazolinyl, pyrroldinyl and imidazolinyl.

The term 'acylamino' includes compounds of the formula 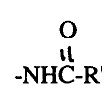 wherein R' is optionally substituted: C₁₋₆alkyl, phenyl, benzyl, heteroaryl, hetroarylC₁₋₄alkyl, or of the formula R⁵⁰-C(=N-OR⁵¹)- wherein R⁵⁰ is phenyl or heteroaryl and R⁵¹ is hydrogen or optionally substituted: C₁₋₆alkyl or benzyl.

In particular 'acylamino' is of the formula R⁵⁰-C(=N-OR⁵¹)- wherein R⁵⁰ is 2-aminothiazol-4-yl, 5-amino-1,2,4-thiadiazol-3-yl or 2-aminooxazol-4-yl and R⁵¹ is C₁₋₆alkyl or carboxyC₁₋₆alkyl.

Examples of substituents for aryl, heteroaryl, heterocyclyl and C₃₋₁₀cycloalkyl include, halo, nitro, cyano, C₁₋₄alkyl, carboxy, amino, hydroxy, C₁₋₄alkylamino, di-(C₁_₄alkyl)amino, C₁₋₄alkoxycarbonyl, sulpho, carbamoyl, C₁₋₄alkylcarbamoyl, di-(C₁₋₄alkyl)carbamoyl, C₁₋₄alkanoyl, C₁₋₄alkoxy, C₁₋₄alkylthio, C₁₋₄alkylsulphinyl and C₁₋₄alkylsulphonyl.

The present invention covers all epimeric, diastereoisomeric and tautomeric forms of the compounds of the formula (I) wherein the absolute stereochemistry at the 7-position is as illustrated in formula (I). When a bond is represented by a wedge, this indicates that in three dimensions the bond would be coming out of the paper and when a bond is hatched, this indicates that in three dimensions the bond would be going back into the paper.

Particular compounds of the present invention are
(7R,8S)-5-acetoxy-8-(1-hydroxyethyl)-9-oxo-1-azabicyclo[5.2.0]non-2,4-diene-2-carboxylic acid;
(7R, 8S)-8-((1R)-1-hydroxyethyl)-5-methylthio-9-oxo-1-azabicyclo[5.2.0]non-2,4-diene-2-carboxylic acid;
(7R,8S)-8-((1R)-1-hydroxyethyl)-5-methanesulphonyl-9-oxo-1-azabicyclo[5.2.0]non-2,4-diene-2-carboxylic acid;
(7R, 8S)-5-(2-aminoethylthio)-8-((1R)-1-hydroxyethyl)-9-oxo-1-azabicyclo[5.2.0]-2,4-diene-2-carboxylic acid; or
(7R, 8S)-5-(2-aminoethylsulphonyl)-8-((1R)-1-hydroxyethyl)-9-oxo-1-azabicyclo[5.2.0]-2,4-diene-2-carboxylic acid; or
pharmaceutically acceptable salts thereof.

Suitable pharmaceutically acceptable salts include acid addition salts such as hydrochloride, hydrobromide, citrate, maleate and salts formed with phosphoric and sulphuric acid. In another aspect suitable salts are base salts such as an alkali metal salt for example sodium or potassium, an alkaline earth metal salt for example calcium or magnesium, an organic amine salt for example triethylamine, morpholine, N-methylpiperidine, N-ethylpiperidine, procaine, dibenzylamine, N,N-dibenzylethylamine or amino acids for example lysine. For the avoidance of doubt there may be one or more salt-forming cations dependent on the number of carboxylic acid functions and the valency of said cations.

Preferred pharmaceutically acceptable salts are sodium and potassium salts. However, to facilitate isolation of the salt during preparation, salts which are less soluble in the chosen solvent may be preferred whether pharmaceutically acceptable or not.

In vivo hydrolysable esters are those pharmaceutically acceptable esters that hydrolyse in the human body to produce the parent compound. Such esters can be identified by administering, eg. intravenously to a test animal, the compound under test and subsequently examining the test animal's body fluids. Suitabe in vivo hydrolysable esters for carboxy include C₁₋₆alkoxymethyl esters for example methoxymethyl, C₁₋₆alkanoyloxymethyl esters for example pivaloyloxymethyl, phthalidyl esters, C₃₋₈cycloalkoxycarbonyloxy-C₁₋₆alkyl esters for example 1-cyclohexyloxycarbonyloxyethyl; 1,3-dioxolen-2-onylmethyl esters for example 5-methyl-1,3-dioxolen-2-onylmethyl; and C₁₋₆alkoxycarbonyloxyethyl esters for example 1-methoxycarbonyloxyethyl and may be formed at any carboxy group in the compounds of this invention. Suitable in vivo hydrolysable ester forming groups for hydroxy include acetyl, propionyl, pivaloyl, C₁₋₄alkoxycarbonyl for example ethoxycarbonyl and phenylacetyl.

In order to use a compound of the formula (I) or a pharmaceutically acceptable salt or in vivo hydrolysable ester thereof for the therapeutic treatment of mammals including humans, in particular in treating infection, it is normally formulated in accordance with standard pharmaceutical practice as a pharmaceutical composition.

Therefore in another aspect the present invention provides a pharmaceutical composition which comprises a compound of the formula (I) or a pharmaceutically acceptable salt or in vivo hydrolysable ester thereof and a pharmaceutically acceptable carrier.

The pharmaceutical compositions of this invention may be administered in standard manner for the disease condition that it is desired to treat, for example by oral, rectal or parenteral administration. For these purposes the compounds of this invention may be formulated by means known in the art into the form of, for example, tablets, capsules, aqueous or oily solutions or suspensions, emulsions, dispersible powders, suppositories and sterile injectable aqueous or oily solutions or suspensions.

The compounds of the present invention may be formulated as dry powder filled vials, which may contain the compound of the present invention alone or as a dry blended mixture. For example an acidic compound of the present invention may be dry blended with an alkali metal carbonate or bicarbonate. Freeze dried formulations of compounds of the present invention, alone or as a mixture with standard excipients, are possible. Standard excipients include structure formers, cryoprotectants and pH modifiers, such as, mannitol, sorbitol, lactose, glucose, sodium chloride, dextran, sucrose, maltose, gelatin, bovine serum albumin (BSA), glycine, mannose, ribose, polyvinylpyrrolidine (PVP), cellulose derivatives, glutamine, inositol, potassium glutamate, erythritol, serine and other amino acids and buffer agents e.g. disodium hydrogen phosphate and potassium citrate.

In addition to the compounds of the present invention the pharmaceutical composition of this invention may also contain, or be co-administered with, one or more known drugs selected from other clinically useful antibacterial agents (for example other beta-lactams or aminoglycosides), inhibitors of beta-lactamase (for example clavulanic acid), renal tubular blocking agents (e.g. probenecid) and inhibitors of metabolising enzymes (for example inhibitors of dehydropeptidases, for example Z-2-acylamino-3-substituted propenoates such as cilastatin) and N-acylated amino acids (for example see EP-A-178911) which reduce adverse effects on the kidney.

A suitable pharmaceutical composition of this invention is one suitable for oral administration in unit dosage form, for example a tablet or capsule which contains between 100 mg and 1 g of the compound of this invention.

A preferred pharmaceutical composition of the invention is one suitable for intravenous, subcutaneous or intramuscular injection, for example a sterile injectable containing between 1 and 50% w/w of the compound of this invention.

Specific examples of compositions, which are constituted as a 1% solution in water, freeze dried and may be made up by adding 0.9% aqueous sodium chloride solution to give the required concentration, preferably 1 mg-10 mg/ml, are as follows:

### Composition 1

| | |
|---|---|
| Compound of Example 1 | 50 mg |

### Compound 2

| | |
|---|---|
| Compound of Example 1 | 50 mg |
| Glycine | 31 mg |

The pharmaceutical compositions of the invention will normally be administered to man in order to combat infections caused by bacteria, in the same general manner as that employed for imipenem due allowance being made in terms of dose levels for the potency and duration of action of the compound of the present invention relative to the clinical use of imipenem. Thus each patient will receive a daily intravenous, subcutaneous or intramuscular dose of 0.05 to 5 g, and preferably 0.1 to 2.5 g, of the compound of this invention, the composition being administered 1 to 4 times per day. The intravenous, subcutaneous and intramuscular dose may be given by means of a bolus injection. Alternatively the intravenous dose may be given by continuous infusion over a period of time. Alternatively each patient will receive a daily oral dose which is approximately equivalent to the daily parenteral dose. Thus a suitable daily dose is 0.05 to 5 g of the compound of this invention, the composition being administered 1 to 4 times uper day.

In a further aspect the present invention provides a process for preparing the compounds of the formula (I) or a pharmaceutically acceptable salt or in vivo hydrolysable ester thereof which process comprises deprotecting a compound of the formula (II): 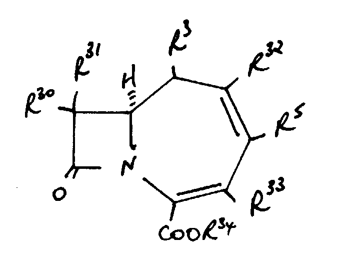 wherein R³ and R⁵ are as hereinabove defined; R³⁰ is R¹ or protected R¹ ; R³¹ is R² or R³⁰ and R³¹ together are optionally substituted C₁₋₆alkylene wherein any optional substituents are optionally protected; R³² is R⁴ or protected R⁴; R³³ is R⁶ or protected R⁶; and -COOR³⁴ is carboxy or protected carboxy; and wherein at least one protecting group is present: and thereinafter if necessary;
(i) forming a pharmaceutically acceptable salt,
(ii) esterifying to form an in vivo hydrolysable ester.

Protecting groups may in general be chosen from any of the groups described in the literature or known to the skilled chemist as appropriate for the protection of the group in question, and may be introduced by conventional methods.

Protecting groups may be removed by any convenient method as described in the literature or known to the skilled chemist as appropriate for the removal of the protecting group in question, such methods being chosen so as to effect removal of the protecting group with minumum disturbance of groups elsewhere in the molecule.

The compounds of the formula II are novel and form another aspect of the invention.

Specific examples of protecting groups are given below for the sake of convenience, in which "lower" signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned is of course within the scope of the invention.

A carboxyl protecting group may be the residue of an ester-forming aliphatic or araliphatic alcohol or of an ester-forming silanol (the said alcohol or silanol preferably containing 1-20 carbon atoms).

Examples of carboxy protecting groups include straight or branched chain (1-12C)alkyl groups (eg isopropyl, t-butyl); lower alkoxy lower alkyl groups (eg methoxymethyl, ethoxymethyl, isobutoxymethyl); lower aliphatic acyloxy lower alkyl groups, (eg acetoxymethyl, propionyloxymethyl, butyryloxymethyl, pivaloyloxymethyl); lower alkoxycarbonyloxy lower alkyl groups (eg 1-methoxycarbonyloxyethyl, 1-ethoxycarbonyloxyethyl); aryl lower alkyl groups (eg p-methoxybenzyl, o-nitrobenzyl, p-nitrobenzyl, benzhydryl and phthalidyl); tri(lower alkyl)silyl groups (eg trimethylsilyl and t-butyldimethylsilyl); tri(lower alkyl)silyl lower alkyl groups (eg trimethylsilylethyl); and (2-6C)alkenyl groups (eg allyl and vinylethyl).

Methods particularly appropriate for the removal of carboxyl protecting groups include for example acid-, base-, metal- or enzymically-catalysed hydrolysis.

Examples of hydroxyl protecting groups include lower alkenyl groups (eg allyl); lower alkanoyl groups (eg acetyl); lower alkoxycarbonyl groups (eg t-butoxycarbonyl); lower alkenyloxycarbonyl groups (eg allyloxycarbonyl); aryl lower alkoxycarbonyl groups (eg benzoyloxycarbonyl, p-methoxybenzyloxycarbonyl, o-nitrobenzyloxycarbonyl, p-nitrobenzyloxycarbonyl); tri lower alkylsilyl (eg trimethylsilyl, t-butyldimethylsilyl) and aryl lower alkyl (eg benzyl) groups.

Examples of amino protecting groups include formyl, aralkyl groups (eg benzyl and substituted benzyl, eg p-methoxybenzyl, nitrobenzyl and 2,4-dimethoxybenzyl, and triphenylmethyl); di-p-anisylmethyl and furylmethyl groups; lower alkoxycarbonyl (eg t-butoxycarbonyl); lower alkenyloxycarbonyl (eg allyloxycarbonyl); aryl lower alkoxycarbonyl groups (eg benzyloxycarbonyl, p-methoxybenzyloxycarbonyl, o-nitrobenzyloxycarbonyl, p-nitrobenzyloxycarbonyl; trialkylsilyl (eg trimethylsilyl and t-butyldimethylsilyl); alkylidene (eg methylidene); benzylidene and substituted benzylidene groups.

Methods appropriate for removal of hydroxy and amino protecting groups include, for example, acid-, base-, metal- or enzymically-catalysed hydrolysis, for groups such as p-nitrobenzyloxycarbonyl, hydrogenation and for groups such as o-nitrobenzyloxycarbonyl, photolytically.

Preferred methods for removal of the p-nitrobenzyl group is hydrogenation using a palladium catalyst or treatment with zinc powder in an aqueous acidic medium.

In another aspect of the present invention the compounds of the formula (I) and (II) may be prepared:
a) for compounds of the formula (I) wherein R⁴ is of the formula -SR⁷ or for compounds of the formula (II) wherein R³² is of the formula -SR^{7'} wherein R^{7'} is R⁷ or protected R⁷, by reacting a compound of the formula HSR^{7'}(compound B) with a compound of the formula (III) 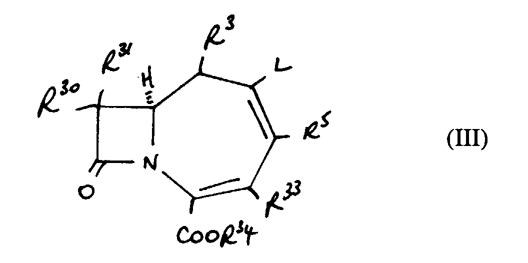 wherein R³, R⁵, R³⁰, R³¹, R³³ and R³⁴ are as hereinabove defined and L is a leaving group;
b) for compounds of the formula (I) wherein R⁴ is of the formula -OR¹⁸ or for compounds of the formula (II) wherein R³² is of the formula -OR^{18'} wherein R^{18'} is R¹⁸ or protected R¹⁸; by reacting a compound of the formula HOR^{18'} (compound D) with a compound of the formula (III) wherein L, R³, R⁵, R³⁰, R³¹, R³³ and R³⁴ are as hereinabove defined;
c) for compounds of the formula (I), wherein R⁴ is of the formula -N(R¹⁹)R²⁰ or for compounds of the formula (II) wherein R³² is of the formula -N(R^{19'})R^{20'} wherein -N(R^{19'})R^{20'} is -N(R¹⁹)R²⁰ or protected -N(R¹⁹)R²⁰:
   (i) by reacting a compound of the formula HN(R^{19'})R^{20'} (compound E) with a compound of the formula (III)
      wherein L, R³, R⁵, R³⁰, R³¹, R³³ and R³⁴ are as hereinabove defined; or
   ii) converting a compound of the formula (IIIA): 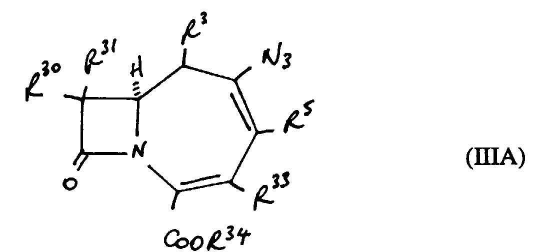 wherein R³, R⁵, R³⁰, R³¹, R³³ and R³⁴ are as hereinbefore defined to a compound of the formula (I) or (II);
d) for compounds of the formula (I), wherein R⁴ is optionally substituted C₁₋₁alkyl, optionally substituted aryl or of the formula -CH₂R²¹ or compounds of the formula (II) wherein R³² is optionally substituted C₁₋₁₀alkyl, optionally substituted aryl, protected substituted C₁₋₁₀alkyl, protected aryl or is of the formula -CH₂R^{21'} wherein R^{21'} is R²¹ or protected R²¹; by reacting a compound
   of the formula R³⁵R³⁶R³⁷SnR³⁸ (compound F) with a compound of the formula (III) wherein L, R³, R⁵, R³⁰, R³¹, R³³ and R³⁴ are as hereinabove defined and R³⁵⁻R³⁷ are C₁₋₆alkyl and R³⁸ is optionally substituted C₁₋₁₀alkyl, aryl or of the formula -CH₂R^{21'};
e) by cyclising a compound for the formula (IV): 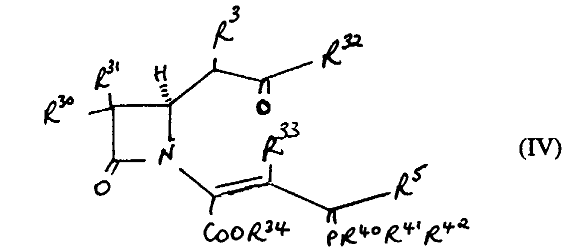 wherein R³,R⁵,R³⁰⁻R³⁴ are as hereinabove defined and R⁴⁰⁻R⁴² are independently selected from C₁₋₆alkoxy, phenyl and phenoxy, wherein any phenyl group is optionally substituted with C₁₋₃alkyl or C₁₋₃alkoxy;
   and wherein any functional group is optionally protected and thereinafter if necessary:
   (i) removing any protecting groups;
   (ii) forming a pharmaceutically acceptable salt;
   (iii) esterifying to form an in vivo hydrolysable ester.

Suitably, in the compound of the formula III, L is the reactive ester of a hydroxy group such as a sulphonate (for example C₁₋₆alkanesulphonyloxy, trifluoromethanesulphonyloxy, benzenesulphonyloxy, toluenesulphonyloxy) or a phosphoric ester (for example a diarylphosphoric ester such as diphenylphosphoric ester). Preferably L is trifluoromethanesulphonyloxy.

The reaction between compounds of the formula (III) and compounds B, D and E may be carried out under conditions known in the carbapenem art for the displacement of a leaving group in the carbapenem with a nucleophile. For example see EP-A-430037, WO 91/14687, EP-A-405774 and US 4530841. In general by reacting the compounds of the formulae (III) with compounds B, D or E in an inert organic solvent in the presence of a base. For example, the reaction between compounds of the formula (III) and B is conveniently performed in a temperature range of -40°C to 50°C, in an inert organic solvent such as tetrahydrofuran, toluene, chloroform, ethyl acetate, N,N-dimethylformamide, acetonitrile or benzene, in the presence of a base such as a tertiary aliphatic amine.

The reaction between compounds of the formula (III) and compounds F is conveniently carried out in an aprotic polar solvent such as tetrahydrofuran, in the presence of a metal halide, such as zinc chloride, a palladium compound, such as Pd₂(DBA).CHCl₃, and a phosphine, such as tri(2,4,6-trimethoxyphenyl)phosphine. For example, in carbapenem art, see EP-A-444889.

The conversion of compound (IIIA) to a compound of the formula (I) or (II) may be carried out under conditions known in the carbapenem art for the conversion of an azide to an amine. For example see US 4310538.

The compounds of the formulae (III), (IIIA) and (IV) are novel and form other aspects of the invention.

The compounds of the formula (IIIA) may be prepared by reacting a compound of the formula (III) with a source of azide, for example HN₃ or NaN₃.

The compounds of the formula (III) may be prepared from a compound of the formula (V): 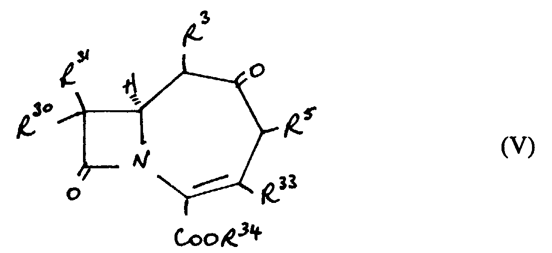 wherein R³, R⁵, R³⁰, R³¹, R³³ and R³⁴ are as hereinabove defined. For example when L is a sulphonate or phosphoric ester, by reacting compounds of the formula (V) with a reactive derivative of L. Suitable reactive derivatives include, for example, an acid anydride such as methane, sulphonic anhydride, trifluoromethanesulphonic anhydride, p-toluenesulphonic anhydride, an acid chloride such as methanesulphonyl chloride, p-toluenesulphonyl chloride or diphenylchlorophosphate.

The reaction is conveniently carried out in an inert solvent, such as methylene chloride, tetrahydrofuran or acetonitrile, in the presence of a base, such as a tertiary aliphatic amine, for example triethylamine, in a temperature range of -78°C to 30°C.

The compound of the formula (V) may be prepared as outlined in Scheme I. 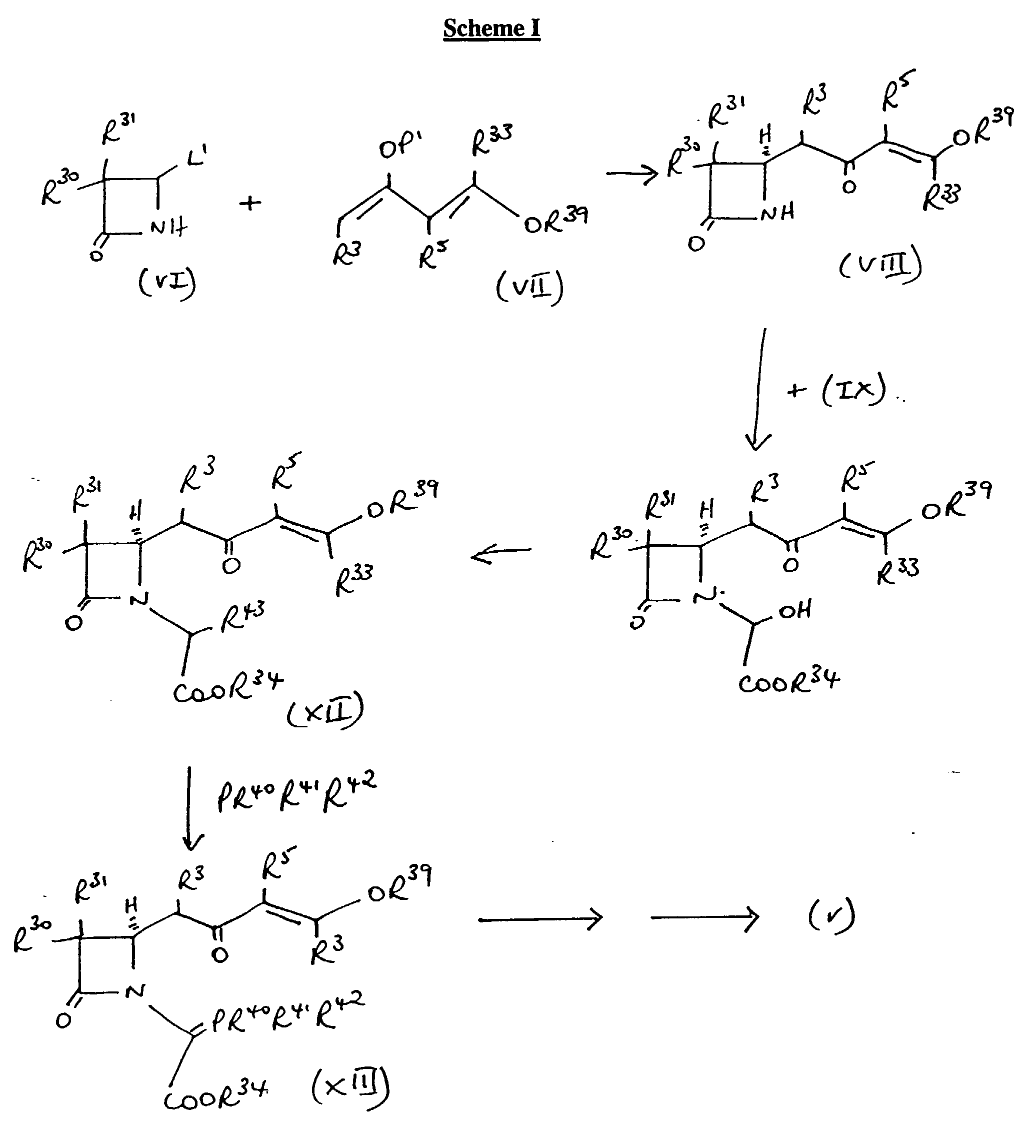 wherein compounds of the formulae (IX) are either of the formula R³⁴OC(O)CHO or R³⁴OC(O)CH(OH)₂ and R³, R⁵, R³⁰, R³¹, R³³, R³⁴ and R⁴⁰⁻R⁴² are as hereinabove defined and R³⁹ is C₁₋₆alkyl, R⁴³ is a halogen atom, L¹ is a leaving group, P¹ is a protecting group and (XI) is a halogenating agent.

The reaction between compounds of the formulae (VI) and (VII) may be carried out in an organic solvent such as dichloromethane, tetrahydrofuran, ether, dioxane or acetone in a temperature range of -100°C to 50°C. A suitable protecting group (P¹) is a silyl protecting group, for example trimethylsilyl. A suitable leaving group is, for example, acetoxy.

The reaction between the compounds of the formulae (VIII) and (IX) is conveniently carried out in a solvent such as acetonitrile, tetrahydrofuran, dioxane or acetone at an elevated temperature, for example 40°C to 100°C, in the presence of a base, such as triethylamine.

Suitable halogenating agents in the conversion of a compound of the formula (X) to a compound of the formula (XII) include N-bromosuccinimide and sulphonylchloride. Suitable conditions are known in the art, for example using an inert organic solvent such as THF, in a temperature range of -30°C to ambient temperature. Compound (XII) may be isolated or converted to the ylide in situ.

The reaction between compounds of the formula PR⁴⁰R⁴¹R⁴² and (XII) is normally carried out in an inert solvent such as acetonitrile, dichloromethane, ethyl ether, tetrahydrofuran or toluene, in a temperature range of 20°C to reflux. The resulting phosphonium compound may be isolated or deprotonated in situ to form a compound of the formula (XIII).

The -C(R⁵)=CR³³(OR³⁹) group in the compounds of the formula (XIII) is a 'masked aldehyde or ketone' which is hydrolysed to an aldehyde or ketone with, for example, paratoluene sulphonic acid monohydrate. Cyclisation of the aldehyde or ketone compound in situ forms a compound of the formula (V). The reaction is conveniently carried out in an inert solvent such as benzene, toluene or xylene in a temperature range of 80°C to 150°C.

Compounds of the formula (IV) may be cyclised under similar conditions to that described for the cyclisation of a compound of the formula (XIII) for example in the presence of paratoluene sulphonic acid monohydrate.

The compounds of the formula (IV) may be prepared from compounds of the formula (XIV): 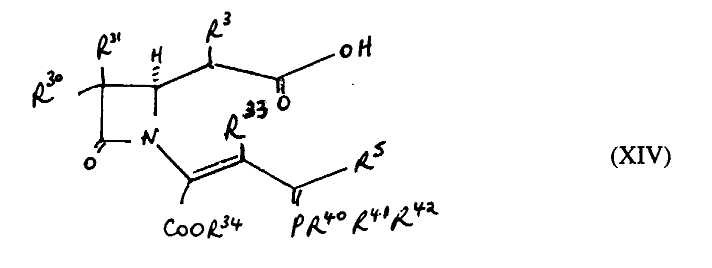 wherein R³, R⁵, R³⁰, R³¹, R³³, R³⁴ and R⁴⁰⁻R⁴² are as hereinabove defined.

For example, when R³² is linked via a carbon atom, the compounds of the formula (XIV) wherein R³⁴ is a protecting group, can be converted to compounds of the formula (IV) by reacting with an activated acylating agent such as 2-pyridyl chlorothioformate and reacting this activated intermediate with a suitable Grignard reagent, such as BrMgR³² under standard conditions known in the art. For example, in an inert solvent such as tetrahydrofuran or ether at 0°C.

Further examples are described for related reactions in US patent 4,260,627, US patent 4,543,257, Tetrahedron, 39, 2531 (1983) and J. Med. Chem. 30, 871 (1987).

The compounds of the formula (XIV) may be prepared as outlined in Scheme II. 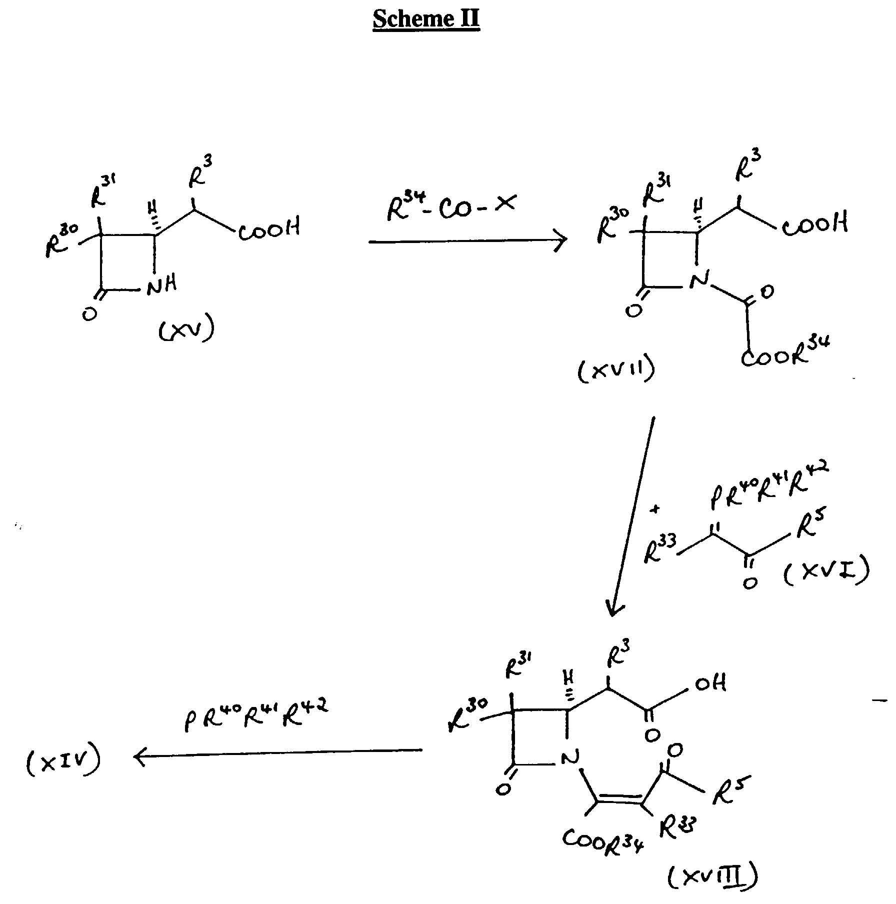 wherein R³, R⁵, R³⁰, R³¹, R³³, R³⁴ and R⁴⁰⁻R⁴² are as hereinabove defined and X is halo.

The reaction between compounds of the formulae (XV) and R³⁴⁻CO-X is conveniently carried out in an inert solvent such as tetrahydrofuran, ethyl acetate or dichloromethane in a temperature range of 0°C to 40°C.

Preferably X is chlorine.

The reaction between compounds of the formulae (XVI) and (XVII) may be carried out under conditions known in the art for Wittig reactions. For example, the conditions described for the cyclisation of the compounds of the formula (VI).

The reaction between the compounds of the formulae PR⁴⁰R⁴¹R⁴² and (XVIII) is conveniently performed under conditions known in the art, for example, in an organic solvent such as toluene, xylene, ethyl acetate, chloroform, dichloromethane, acetonitrile or dimethylformamide. Typically the reaction is carried out at an elevated temperature for example 60-150°C.

For examples of suitable conditions see EP-A-394991. The following biological test methods, data and Example serve to illustrate the present invention.

### Antibacterial Activity

The pharmaceutically acceptable carbapenem compounds of the present invention are useful antibacterial agents having a broad spectrum of activity in vitro against standard-laboratory microorganisms, both Gram-negative and Gram-positive, which are used to screen for activity against pathogenic bacteria. The antibacterial spectrum and potency of a particular compound may be determined in a standard test system. In particular the carbapenems of the present invention show good stability to beta-lactamases and have a particularly good elimination half life in mammals. In general compounds show significant improvement over imipenem.

The antibacterial properties of the compounds of the invention may also be demonstrated in vivo in conventional tests.

Carbapenem compounds have generally been found to be relatively non-toxic to warm-blooded animals, and this generalisation holds true for the compounds of the present invention. Compounds representative of the present invention were administered to mice at doses in excess of those required to afford protection against bacterial infections, and no overt toxic symptoms or side effects attributable to the administered compounds were noted.

The following results were obtained for representative compounds on a standard in vitro test system using Diagnostic Sensitivity Test. The antibacterial activity is described in terms of the minimum inhibitory concentration (MIC) determined by the agar-dilution technique with an inoculum size of 104 CFU/spot.

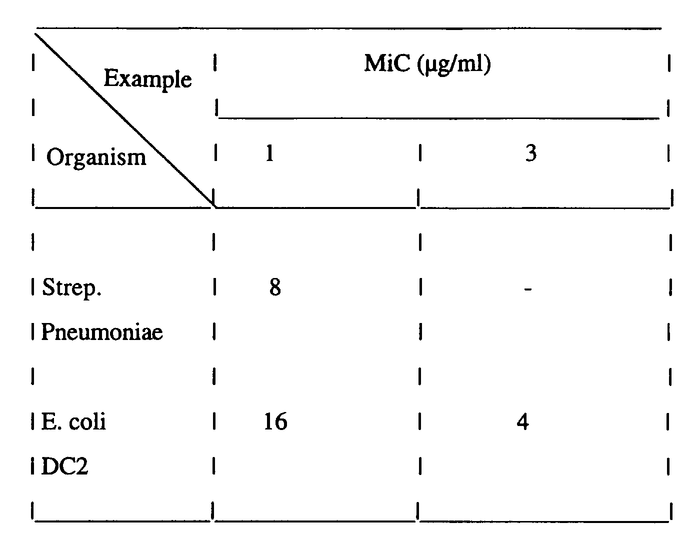

In the examples:
(a) THF means tetrahydrofuran; and
(b) Evaporation of solvents was carried out under reduced pressure.

### Example 1

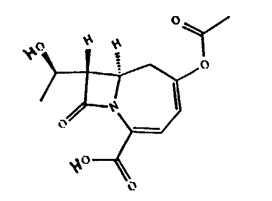

### Method A:

### (7R,8S)-5-Acetoxy-8-(1-hydroxyethyl)-9-oxo-1-azabicyclo[5.2.0]non-2.4-diene-2-carboxylic acid.

4-Methoxybenzyl (7R,8S)-5-acetoxy-8-[(1R)-1-hydroxyethyl]-9-oxo-1-azabicyclo[5.2.0]non-2,4-diene-2-carboxylate (29 mg, 0.07 mM) was dissolved in dichloromethane (3 ml) and cooled to -78°C. AlCl₃ (6 mg, 0.04 mM) was added and reaction took place instantaneously. Phosphate buffer (3 ml, PH7) was added and the mixture allowed to warm to ambient temperature. The aqueous phase was isolated, the solvent removed and the residue purified by subjecting to chromatography on HP20SS resin (10 ml), eluting with water. The appropriate fractions were lyophilised to give (7R,8S)-5-acetoxy-8-[(1R)-hydroxyethyl]-9-oxo-1-azabicyclo[5.2.0]non-2,4-diene-2-carboxylic acid (11 mg, 50%).
Ms (-ve FAB) 280 (M-H)⁺
Nmr: (90 MHz, D₂O): δ 1.46 (d, 3H); 2.37 (s, 3H); 3.07-3.21 (m, 3H); 3.87 (m, 1H); 5.89 (d, 1H); 6.02 (d, 1H).

The starting material was prepared as follows:

(3R,4R) 4-acetoxy-3-[(1R)-(1-(tert-butyldimethylsilyloxy)ethyl)]azetidin-2-one (2 g, 56.96 mM) was dissolved in dichloromethane (150 ml) at ambient temperature and 4-methoxy-2-trimethylsilyloxybut-1,3- diene (1.5 ml, 7.65 mM) added followed by zinc iodide (2.2 g, 17 mM). The reaction mixture was shielded from light and stirred for 15 hours. The mixture was then washed with a 5% aqueous solution of sodium bicarbonate (30 ml) and then water (30 ml). The organic phase was dried with magnesium sulphate and the solvent evaporated. The residue was purified by chromatography on silica, eluting with ethyl acetate/ dichloromethane (1:1) to to give (3S,4R) 3-((1R)-1-(tert-butyldimethylsilyloxy)ethyl)-4-(4-methoxy-2-oxobut-3-en-1-yl)azetidin-2-one (0.96 g, 43%).
Nmr: (90 MHz, CDCl₃): δ 0.07 (s, 7H); 0.87 (s, 9H); 1.22 (d, 3H); 2.45-3.10 (m, 3H); 3.73 (s, 3H); 3.90-4.30 (m, 2H); 5.57 (d, 1H); 6.05 (brs, 1H); 7.60 (d, 1H).

A mixture of (3S,4R) 3-((1R)-1-(tert-butyldimethylsilyloxy)ethyl)-4-(4-methoxy-2-oxobut-3-en-1-yl)azetidin-2-one (100 mg, 0.3 mM), 4-methoxybenzyl dihydroxyacetate (72 mg, 0.33 mM) and 2 drops of triethylamine in benzene (5 ml) was heated at reflux for 1 hour. The solvent was evaporated and the product purified by subjecting to chromatography on silica, eluting with ether to give 4-methoxybenzyl 2-[(3S,4R)-3-[(1R)-1-(tert-butyldimethylsilyloxy)ethyl]4-[4-methoxy-2-oxobut-3-en-1-yl]-2-azetidinone-1-yl]-2-hydroxy- acetate (147 mg, 93%). IR (film, Nacl) 3310, 1745.

4-Methoxybenzyl 2-[(3S,4R)-3-[(1R)-1-(tert-butyldimethylsilyloxy)ethyl]-4-[4-methoxy-2-oxobut-3-en-1-yl]-2-azetidinone-1-yl]-2-hydroxyacetate (4.16 g, 8 mM) was dissolved in THF (150 ml) and cooled to -20°C. 2,6-Lutidine (3 ml, 24 mM) was added, then thionyl chloride (0.9 ml, 12 mM). The mixture was allowed to warm to ambient temperature over 30 minutes. The mixture was filtered, the solvent evaporated, the residue redissolved in benzene, filtered and the solvent evaporated. The residue was redissolved in THF (150 ml) and triphenylphosphine (4.2 g, 16 mM), 2,6-lutidine (2 ml, 16 mM) and sodium iodide (0.25 g, 1.6 mM) added successively. The mixture was stirred for 15 hours at ambient temperature, after which it was filtered, evaporated and purified by subjecting to chromatography on silica, eluting with ethyl acetate/cyclohexane (1:1) to give 4-methoxybenzyl 2-[(3S,4R)-3-[(1R)-1-(tertbutyldimethylsilyloxy)ethyl]-4-[4-methoxy-2-oxobut-3-en-1-yl]-2-azetidinon-1-yl]-2-(triphenylphosphoronylidene)acetate (4.25 g, 66.4%). IR (film, NaCl) 3450, 1730

Toluenesulphonic acid (4 mg, 0.03 mM) was added to 4-methoxybenzyl 2-[(3S,4R)-3-[(1R)-1(tertbutyldimethylsilyloxy)ethyl]-4-[4-methoxy-2-oxobut-3-en-1-yl]-2-azetidinon-1-yl]-2-(triphenylphosphoronylidene)acetate (240 mg, 0.3 mM) in toluene (10 ml) and the mixture warmed to 50°C. After 3 hours, the mixture was diluted with ethyl acetate (40 ml) and washed with a 5% aqueous solution of sodium bicarbonate (20 ml). The organic phase was dried with magnesium sulphate, the solvent evaporated and the residue purified by subjecting to chromatography on a silica column (20 ml), eluting with ethyl acetate/dichloromethane to give 4-methoxybenzyl (7R,8S)-8-[(1R)-1-(tert-butyldimethylsilyloxy)ethyl]-5,9-dioxo-1-azabicyclo[5.2.0]non-2-ene-2-carboxylate (105 mg, 76.6%).
Nmr: (90 MHz, CDCl₃): δ 0.05 (s, 3H); 0.07 (s, 3H); 0.86 (s, 9H); 1.26 (d, 3H); 2.65-3.00 (m, 3H); 3.35 (dd, 2H); 3.80 (s, 3H); 3.90-4.35 (m, 2H); 5.18 (brs, 2H); 6.10 (t, 1H); 6.80-6.95 (m, 2H); 7.23-7.40 (m, 2H).

Triethylamine (77 µl, 0.55 mM) followed by acetyl chloride (23 µl, 0.3 mM) were added to a solution of 4-methoxybenzyl (7R,8S)-8-[(1R)-1-(tert-butyldimethylsilyloxy)ethyl]-5,9-dioxo-1-azabicyclo [5.2.0]non-2-ene-2-carboxylate (120 mg, 0.25 mM) in dichloromethane (15 ml) cooled with an ice bath. The reaction occurred immediately. The solvent was evaporated and the residue purified by chromatography on a silica column, eluting with ethyl acetate/cyclohexane (1:3) to give 4-methoxybenzyl (7R,8S)-5-acetoxy-8-[(1R)-1-ter(t-butyldimethylsilyloxy)ethyl]-9-oxo- 1 -azabicyclo[5.2.0]non-2,4-diene-2-carboxylate (100 mg, 78%).
Nmr: (300 MHz, CDCl₃): δ 0.06 (s, 3H); 0.07 (s, 3H); 0.85 (s, 9H); 1.28 (d, 3H); 2.15 (s, 3H); 2.77 (dd, 1H); 2.84-2.87 (m, 2H); 3.67 (m, 1H); 3.80 (s, 3H); 4.30 (m, 1H); 5.21 (s, 2H); 5.75 (d, 1H); 6.08 (d, 1H); 6.88 (d, 2H); 7.35 (d, 2H).
¹³C (300 MHz, CDCl₃): δ -4.99, -4.47, -0.09, 17.76, 20.73, 22.72, 25.63, 40.43, 47.99, 55.16, 65.86, 67.24, 112.11, 113.03, 113.29, 127.66, 128.21, 130.35, 153.15, 159.63, 162.24, 163.54, 169.01.

To a solution of 4-methoxybenzyl (7R,8S)-5-acetoxy-8-[(1R)-1-(tert-butyldimethylsilyloxy)ethyl]-9-oxo- 1-azabicyclo[5.2.0]non-2,4-diene-2-carboxylate (88 mg, 0.17 mM) in methanol (6 ml) was added hydrochloric acid (10 drops, 2N). After 6 hours the solvent was evaporated and the product purified by chromatography on silica (10 ml), eluting with ethyl acetate/dichloromethane, (1:1) to give 4-methoxybenzyl (7R,8S)-5-acetoxy-8-[(R)-1-hydroxyethyl]-9-oxo-1-azabicyclo[5.2.0]non-2,4-diene-2-carboxylate (29 mg, 39%).
Nmr: (90 MHz, CDCl₃): δ 1.32 (d, 3H); 2.14 (s, 3H); 2.80-2.97 (m, 3H); 3.70 (m, 1H); 3.79 (s, 3H); 4.33 (m, 1H); 5.18 (s, 2H); 5.74 (d, 1H); 6.13 (d, 1H); 6.87 (d, 2H); 7.32 (d, 2H). MS (+ve FAB) 402 (M+H)⁺

### Method B:

4-Nitrobenzyl(7R,8S)-5acetoxy-8-[(1R)-1-hydroxyethyl]-9-oxo-1-azabicyclo[5.2.0] non-2,4-diene-2-carboxylate (150mg, 0.36mmol) was dissolved in a 1:1 (v/v) mixture of THF and water (25ml) at ambient temperature. The pH of the solution was adjusted with a pHstat to 3.6 and zinc powder (285mg, 4.3mmol) was added portion wise to the solution while the pH of the solution was maintained at 3.6 with 2N HCl. After 15 min, the pH was adjusted to 6.5, the suspension filtered over Celite and the THF evaporated in vacuo. The remaining aqueous solution was purified by column chromatography on a reverse phase silica gel (Nucleosil C18 10 µ, 3.5 x 20 cm) with water as eluant. Lyophilisation of the appropriate fractions yielded
(7R, 8S)-5 Acetoxy-8-[(1R)-1-hydroxyethyl]-9-oxo-1-azabicydo[5.2.0]-non-2,4-diene-2-carboxylic acid, sodium salt (61mg, 56%).
1H NMR (400 MHz, D2O) δ:1.31 (d, 3H); 2.22 (s, 3H); 2.94-2.96 (m, 2H); 3.01 (dd, 1H); 3.77 (m, 1H); 4.36 (m, 1H); 5.77 (d, 1H); 5.84 (d, 1H).
MS (ESI) : 303 (MNa)+

The starting material was prepared as follows:

A mixture of (3S,4R) 3-[(1R)-1-(tert-butyldimethylsilyloxy)ethyl]4-[(4Z)-methoxy-2-oxobut-3-en-1-yl]-2-azetidinone (10g, 30mmol), 4-nitrobenzyl-1,1-dihydroxyacetate (7.2g, 34mmol) and triethylamine (1.6ml, 11mmol) in toluene (500ml) was heated at reflux in a Dean-Stark apparatus in the presence of molecular sieves (4A°) for 6 hours. Additional 4-nitrobenzyl-1,1-dihydroxyacetate (1.5g, 7mmol) was added during the reaction. The solvent was evaporated
and the residue purified by flash chromatography on silica using acetonitrile/dichloromethane (1:9) as eluant to give 4-nitrobenzyl
2-[(3S,4R)-3-[(1R)-1-(tert-butyldimethylsilyloxy)ethyl]-4-[(4Z)methoxy-2-oxobut-3-en-1-yl]-2-azetidinon-1-yl]-2-hydroxyacetate as two diastereoisomers (8.5g, 53%).

### *Less polar diastereoisomer :

1H NMR (400 MHz, CDCl3) δ:0.04(s, 3H); 0.06 (s, 3H); 0.85 (s, 9H); 1.21 (d, 3H); 2.81-2.96 (m, 3H); 3.70 (s, 3H); 4.15-4.21 (m, 2H); 5.18 (d, 1H); 5.23 (d, 1H); 5.44 (d, 1H); 5.46 (d, 1H); 5.59 (d, 1H); 7.53 (d, 2H); 8.21 (d, 2H).

### *More polar diastereoisomer :

1H NMR (400 MHz, CDCl3) δ:0.03 (s, 6H); 0.83 (s, 9H); 1.24 (d, 3H); 2.82 (dd, 1H); 2.84 (d, 1H); 3.0 (dd, 1H); 3.75 (s, 3H); 4.06-4.10 (m, 2H); 4.74 (d, 1H); 5.33 (d, 1H); 5.38 (d, 1H); 5.58 (d, 1H); 5.60 (d, 1H); 7.58 (d, 2H); 7.62 (d, 1H); 8.23 (d, 2H).

To a solution of 4-nitrobenzyl 2-[(3S,4R)-3-[(1R)-1(tert-butyldimethylsilyloxy)ethyl]4-[(4Z)-methoxy-2-oxobut-3-en-1-yl]-2-azetidinon-1-yl]-2-hydroxyacetate (8.49g, 15.8mmol) in THF (300ml) at -20°C was added successively 2,6-lutidine (5.5ml, 47.5mmol) and thionylchloride (1.7ml, 24mmol). The cooling bath was removed and the reaction mixture stirred at ambient temperature for 30 minutes. The mixture was filtered, the solvent evaporated, the residue redissolved in toluene, the solution again filtered and evaporated. The residue was taken up in THF (300ml) and triphenylphosphine (8.3 g, 32 mmol), 2,6-lutidine (3.7ml, 32 mmol) and sodium iodide (0.5g, 32 mmol) were added successively. The reaction mixture was stirred at ambient temperature for 1 hour, filtered and evaporated. The residue was purified by flash chromatography on silica using dichloromethane / ethyl acetate (8:2) as eluant to afford the title compound 4-nitrobenzyl 2-[(3S,4R)-3[(1R)-1-(tert-butyldimethylsilyloxy)ethyl]4-[(4Z)-methoxy-2-oxobut-3en- 1-yl]-2-azetidinon-1-yl]-2-(triphenylphosphoranylidene)acetate (7.5g, 58%).
1H NMR (400 MHz, CDCl3) δ:-0.13 (s, 2H); -0,06 (s, 3H); 0.76 (s, 9H); 1.10 (d, 3H); 2.52-2.67 (m, 2H); 2.88-3.09 (m, 2H); 3.18-3.40 (m, 1H); 3.65 (s, 3H); 4.72-5.76 (complex pattern, 3H); 6.68 (d, 1H); 7.43-8.25 (m, 19 H).

A solution of 4-nitrobenzyl 2-[(3S,4R)-3-[(1R)-1(tert-butyldimethylsilyloxy)ethyl]4-[(4Z)-methoxy-2-oxobut-3-en-1-yl]-2-azetidinon-1-yl]-2-(triphenylphosphoranylidene)acetate (6.9g, 8.4 mmol) and para toluenesulphonic acid monohydrate (0.56g, 2.9 mmol) in toluene (280ml) was heated at 80°C for 10 minutes and then stirred at 60°C for 5 hours. Additional para toluenesulphonic acid monohydrate (0.51g, 2.7 mmol) was added during the reaction.

The solvent was removed under reduced pressure, the residue was taken up in ethyl acetate, washed with a 5% aqueous solution of NaHCO3 and water. The organic phase was dried over magnesium sulphate, the solvent evaporated and the residue purified by flash chromatography on a silica gel column, eluting with dichloromethane / ethyl acetate (85:15) to afford 4-nitrobenzyl (7R,8S)-8-[(1R)-1-(tert-butyldimethylsilyloxy)ethyl]-5,9-dioxo-1-azabicyclo[5.2.0]non-2-ene-2-carboxylate (1.67g, 41%).
1H NMR (400 MHz, CDCl3) δ:0.04 (s, 3H); 0.07 (s, 3H); 0.85 (s, 9H); 1.26 (d, 3H); 2.93-3.01 (m, 3H); 3.41 (ddd, 1H); 4.06 (m, 1H); 4.21 (m, 1H); 5.30 (d, 1H); 5.37 (d, 1H); 6.18 (dd, 1H); 7.57 (d, 2H); 8.22 (d, 2H).
MS (ESI) : 511(MNa+).

Triethylamine (1.1ml, 7.5mmol) followed by acetyl chloride (0.295ml, 4.1mmol) were added to a solution of 4-nitrobenzyl (7R,8S)-8-[(1R)-1-(tert-butyldimethylsilyloxy)ethyl]-5,9-dioxo-1-azabicyclo[5.2.0]non-2-ene-2-carboxylate (1.67g, 3.4mmol) in anhydrous dichloromethane (220ml) cooled with an ice bath. After 5 minutes, the reaction mixture was washed with water and dried over magnesium sulphate. The solvent was evaporated and the residue purified by flash chromatography, eluting with dichloromethane / ethyl acetate (95:5) to give 4-nitrobenzyl (7R,8S)-5-acetoxy-8-[(IR)-1-(tenbutyldimethylsilyloxy)ethyl]-9-oxo-1-azabicydo[5.2.0]non-2,4-diene-2-carboxylate (1.4g, 77%).
1H NMR (400 MHz, CDCl3) δ:0.04 (s, 3H); 0.07 (s, 3H); 0.85 (s, 9H); 1.26 (d, 3H); 2.93-3.01 (m, 3H); 3.37 (dd, 1H); 3.45 (dd, 1H); 4.06 (m, 1H); 4.21 (m, 1H); 5.31 (d, 1H); 5.37 (d, 1H); 6.18 (dd, 1H); 7.57 (d, 2H); 8.22 (dd, 2H).

To a solution of 4-nitrobenzyl (7R,8S)-5-acetoxy-8-[(1R)-1-(tert-butyldimethylsilyloxy)ethyl]-9-oxo-1-azabicyclo[5.2.0]non-2,4-diene-2-carboxylate (1.35g, 2.5mmol) in anhydrous THF (75ml) was added under argon successively acetic acid (2.18ml, 38mmol) and a 1.1M THF solution of N-tetrabutylammonium fluoride (12ml, 12.7mmol). The reaction mixture was stirred at ambient temperature for 3 days. Additional acetic acid (0.44ml, 7.6mmol) and N-tetrabutylammonium fluoride (2.4ml, 25mmol) were added after 2 days. THF was evaporated, the residue taken up in ethyl acetate and the solution was successively washed with a 5% aqueous solution of NaHCO3 and water. The organic phase was dried over magnesium sulphate, the solvent evaporated and the residue purified by flash chromatography on a silica gel column, eluting with dichloromethane / ethyl acetate (1:1) to afford 4-nitrobenzyl (7R,8S)-5 acetoxy-8-[(1R)-hydroxethyl]-9-oxo-1-azabicyclo[5.2.0] non-2,4-diene-2-carboxylate as a foam (0.5g, 47%).
1H NMR (400 MHz, CDCl3) δ:1.36 (d, 3H); 2.18 (s, 3H); 2.86 (dd, 1H); 2.92-2.94 (m, 2H); 3.72 (m, 1H); 4.35 (m, 1H); 5.35 (s, 2H); 5.82 (dd, 1H); 6.22 (d, 1H); 7.59 (d, 2H); 8.23 (d, 2H).
MS (ESI) : 439 (MNa)+

**Example 2**

(7R, 8S)-8-[(1R)-1-Hydroxyethyl]-5-methylthio-9-oxo-1-azabicyclo [5.2.0]non-2.4-diene-2-carboxylic acid, sodium salt :

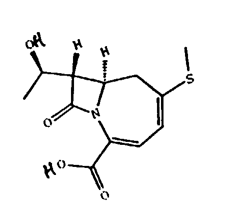

To a solution of 4-nitrobenzyl (7R,8S)-8-[(1R)-1-hydroxyethyl]-5-methylthio-9-oxo-1-azabicyclo [5.2.0] non-2,4-diene-2-carboxylate (179 mg, 0.44 mmol) in DMF (45 ml) was added 10 % palladium on charcoal (80 mg). The reaction mixture was stirred under a hydrogen atmosphere for 1.5 hours. The catalyst was filtered off, the organic phase partially concentrated and purified by chromatography on HP20SS resin (60 ml) with a gradient of CH3CN (0 to 50 %) in water as mobile phase. Freeze drying of the appropriate fractions gave the expected acid (80 mg) only partially purified. The solid was suspended in water (6 ml) and solubilisised by adjusting the pH of the solution to 7.3 with an aqueous saturated solution of NaHCO3. The mixture was then purified by reverse phase chromatography (Nucleosil C18 10 µ, 3.5 x 20 cm) with water as eluant to give, after freeze drying, the title compound (36 mg, 32 % yield) as a yellow solid.
1H NMR (400 MHz, D2O) δ:1.32 (d, 3H); 2.34 (s, 3H); 2.82 (ddd, 1H); 2.94 (d, 1H); 2.99 (dd, 1H); 3.72 (ddd, 1H); 4.35 (m, 1H); 5.66 (brd, 1H); 5.96 (d, 1H).
MS (FAB + ve) : 292 (MNa)+

The starting materials were prepared as follows :

To a solution of 4-nitrobenzyl (7R, 8S)-8-[(1R)-1-(tert-butyldimethylsilyloxy)ethyl]-5,9-dioxo-1-azabicyclo[5.2.0]non-2-ene-2-carboxylate (1.5 g, 3.07 mmol) in acetonitrile (15 ml) was added at 0°C N,N-diisopropylethylamine (1.1ml, 6.45mmol). The reaction mixture was cooled at -40°C and trifluoromethanesulfonic anhydride (0.57 ml ; 3.38 mmol) was added. After 5 minutes, further N,N-diisopropylethylamine (1.1ml, 6.45mmol) was added to the reaction mixture. Methylmercaptan was then bubbled through the solution at -40°C during a few seconds. The mixture was allowed to warm to ambient temperature over 1.5 hours. The solvent was evaporated and the residue purified by chromatography on silica gel eluting with ethyl acetate / dichloromethane 3 : 97 to give 4-nitrobenzyl (7R, 8S)-8-[(1R)-1-(tert-butyldimethylsilyloxy)ethyl]5-methylthio-9-oxo-1-azabicyclo[5.2.0]non-2,4-diene-2-carboxylate (776 mg, 47 %).
1H NMR (CDCl3) δ:0.03 (s, 3H); 0.07 (s, 3H); 0.84 (s, 9H); 1.28 (d, 3H); 2.32 (s, 3H); 2.69 (ddd, 1H); 2.78 (dd, 1H); 2.87 (d, 1H); 3.61 (brd, 1H); 4.29 (m, 1H); 5.30 (d, 1H); 5.38 (d, 1H); 5.52 (brd, 1H); 6.29 (d, 1H); 7.60 (d, 2H); 8.21 (d, 2H).
MS (ESI): 541 (MNa)+.

To a solution of 4-nitrobenzyl (7R,8S)-8-[(1R)-1-(tert-butyldimethylsilyloxy)ethyl]-5-methylthio-9-oxo-1-azabicyclo [5.2.0] non-2,4-diene-2-carboxylate (213 mg, 0.41 mmol) in anhydride THF (8 ml) was added acetic acid (0.35 ml) and a 1.1 M solution in tetrahydrofuran of tetrabutylammonium fluoride (1.9 ml ; 2.04 mmol). After stirring for 3 days at ambient temperature, the reaction mixture was diluted with ethyl acetate (20 ml) and then washed with an 5 % aqueous solution of NaHCO3 and water. After evaporation of the solvents, the residue was taken up with methylene chloride. The insoluble material was filtered off and identified as 4-nitrobenzyl (7R,85)-8-[(1R)-1-hydroxyethyl]-5-methylthio-9-oxo-1-azabicyclo[5.2.0]non-2,4-diene-2-carboxylate :

The filtrate was concentrated and the residue was purified by chromatography on silica gel eluting with ethyl acetate / dichloromethane 3:7 to give an other batch of the 4-nitrobenzyl (7R, 8S)-8-[(1R)-1-hydroxyethyl]-5-methylthio-9-oxo-1-azabicyclo-[5.2.0]non-2,4-diene-2-carboxylate (total=102 mg, 62 % overall yield).
1H NMR (400 MHz, CDCl3) δ:1.37 (d, 3H); 1.87 (d, 1H); 2.33 (s, 3H); 2.74 (ddd, 1H); 2.85 (dd, 1H); 2.93 (d, 1H); 3.64 (dt, 1H); 4.34 (m, 1H); 5.32 (d, 1H); 5.37 (d, 1H); 5.54 (brd, 1H); 6.35 (d, 1H); 7.60 (d, 2H); 8.22 (d, 2H).
MS (ESI) : 427 (MNa)+.

**Example 3**

(7R, 8S)-8-[(1R)-1-Hydroxyethyl]-5-methanesulphonyl-9-oxo-1-azabicyclo[5, 2.01non-2,4-diene-2-carboxylic acid, sodium salt:

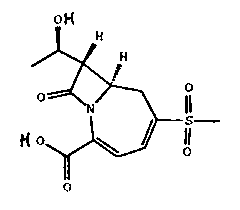

The title compound was prepared from 4-nitrobenzyl (7R,8S)-8-[(1R)-1-hydroxyethyl]-5-methanesulphonyl-9-oxo-1-azabicyclo [5.2.0] non-2,4-diene-2-carboxylate by the procedure described in Example 1, method B. 1H NMR (400 MHz, D2O) δ:1.34 (d, 3H); 2.90 (ddd, 1H); 3.15 (s, 3H); 3.27 (dd, 1H); 3.49 (d, 1H); 3.82 (m, 1H); 4.38 (m, 1H); 5.78 (d, 1H); 7.10 (dd, 1H).
MS (ESI) : 346 (MNa)+

The starting materials were prepared as follows :

3-Chloroperoxybenzoic acid at (50% pure, 0.89g, 2.58mmol) was added at 0°C to a solution of 4-nitrobenzyl (7R,8S)-8-[(1R)-1-(tert-butyldimethylsilyloxy)ethyl]-5-methylthio-9-oxo-1-azabicyclo[5.2.0]non-2,4-diene-2-carboxylate (0.67g, 1.29mmol) in dichloromethane (17ml). The reaction mixture was stirred at 0°C for 3hours. The solution was washed with an 5% aqueous solution of NaHCO3, water and dried over magnesium sulphate. The solvent was evaporated and the residue purified by flash chromatography, eluting with dichloromethane/ethyl acetate (85:15) to give 4-nitrobenzyl (7R, 8S)-8-[(1R)-1-(tert-butyldimethylsilyloxy)ethyl]-5-methanesulphonyl-9-oxo-1-azabicyclo[5.2.0]non-2,4-diene-2-carboxylate (0.48g, 67%).
1H NMR (400 MHz, CDC13) δ:0.02 (s, 3H); 0.08 (s, 3H); 0.83 (s, 9H); 1.28 (d, 3H); 2.64 (ddd, 1H); 2.94 (s, 3H); 2.99 (dd, 1H); 3.56 (d, 1H); 3.73 (m, 1H); 4.34 (m, 1H); 5.38 (s, 2H); 6.14 (d, 1H); 7.14 (dd, 1H); 7.59 (d, 2H); 8.23 (d, 2H).
MS (ESI) : 573 (MNa)+

4-Nitrobenzyl (7R, 8S)-8-[(1R)-1-hydroxyethyl]-5-methanesulphonyl-9-oxo-1-azabicyclo [5.2.0]non-2,4-diene-2-carboxylate was prepared from 4-nitrobenzyl (7R, 8S)-8-[(1R)-1-(tert-butyldimethylsilyloxy)ethyl]-5-methanesulphonyl-9-oxo-1-azabicyclo [5.2.0]non-2,4-diene-2-carboxylate by a similar procedure to that described in Example 1, method B.
1H NMR (400 MHz, CDCl3): δ:1.38 (d, 3H); 2.00 (d, 1H); 2.69 (ddd, 1H); 2.95 (s, 3H); 3.04 (dd, 1H); 3.62 (dd, 1H); 3.73 (m, 1H); 4.38 (m, 1H); 5.38 (s, 2H); 6.22 (d, 1H); 7.16 (dd, 1H); 7.59 (d, 2H); 8.23 (d, 2H).
MS (ESI) : 459 (MNa)+; 436 (MH)+.

**Example 4**

(7R,8S)-5-(2-Aminoethylthio)-8-[(1R)-1-hydroxyethyl]-9-oxo-1-azabicyclo [5,2.0]non-2,4-diene-2-carboxylic acid:

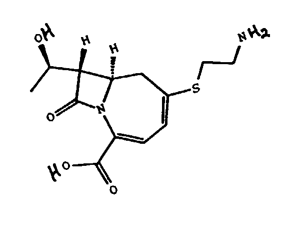

The title compound was prepared from 4-nitrobenzyl (7R, 8S)-8-[(1R)-1-hydroxyethyl]-5-[2-(4-nitrobenzyloxycarbonyl)- aminoethylthio-9-oxo-1-azabicyclo [5.2.0]non-2,4-diene-2-carboxylate by a similar procedure to that described in Example 1, method B.
1H NMR (400 MHz, D2O): δ1.32 (d, 3H); 2.83 (ddd, 1H); 3.00-3.05 (m, 2H); 3.08-3.20 (m, 2H); 3.22-3.31 (m, 2H); 3.72 (m, 1H); 4.35 (m, 1H); 5.86 (d, 1H); 5.98 (m, 1H).
MS (ESI): 321 (MH)+

The starting materials were prepared as follows :

4-Nitrobenzyl (7R, 8S)-8-[(1R)-1-(tert-butyldimethylsilyloxy)ethyl]-5-[2-(4-nitrobenzyloxycarbonyl)-aminoethylthio]-9-oxo-1-azabicyclo[5.2.0]non-2,4-diene-2-carboxylate was prepared from 4-nitrobenzyl (7R, 8S)-8-[(1R)-1-(tert-butyldimethylsilyloxy)ethyl]-5,9-dioxo-1-azabicyclo[5.2.0]non-2-ene-2-carboxylate by using a similar procedure to that described in Example 2 except 2-[4-nitrobenzyloxycarbonylamino]ethanethiol was used instead of methylmercaptan.
1H NMR (400 MHz, CDCl3) δ:0.03 (s, 3H); 0.07 (s, 3H); 0.84 (s, 9H); 1.27 (d, 3H); 2.67 (ddd, 1H); 2.79 (dd, 1H); 2.88 (d, 1H); 2.97 (t, 2H); 3.45 (d, 1H); 3.49 (d, 1H); 3.61 (d, 1H); 4.29 (m, 1H); 5.20 (brs, 2H); 5.31 (d, 1H); 5.38 (d, 1H); 5.80 (d, 1H); 6.24 (d, 1H); 7.50 (d, 2H); 7.60 (d, 2H); 8.20-8.23 (m, 4H).
MS (ESI) : 727 (MH)+

4-Nitrobenzyl (7R, 8S)-8-[(1R)-1-hydroxyethyl]-5-[2-(4-nitrobenzyloxycarbonyl)aminoethylthio]-9-oxo-1-azabicyclo[5.2.0]non-2,4-diene-2-carboxylate was prepared from 4-nitrobenzyl (7R, 8S)-8-[(1R)-1-(tert-butyldimethylsilyloxy)]ethyl]-5-[2-(4-nitrobenzyloxycarbonyl)aminoethyl]thio-9-oxo-1-azabicyclo[5.2.0]non-2,4-diene-2-carboxylate by a similar procedure described in Example 1, method B.
1H NMR (400 MHz, CDCl3): δ1.37 (d, 3H); 2.71 (ddd, 1H); 2.85 (dd, 1H); 2.91-3.02 (m, 3H); 3.40-3.49 (m, 2H); 3.69 (d, 1H); 4.33 (m, 1H); 5.19 (brs, 2H); 5.32 (d, 1H); 5.37 (d, 1H); 5.90 (d, 1H); 6.26 (d, 1H); 7.50 (d, 2H); 7.59 (d, 2H); 8.22 (d, 4H).

**Example 5**

(7R. 8S)-5-(2-Aminoethylsulphonyl)-8-[(1R)-1-hydroxyethyl]-9-oxo-1-azabicyclo[5.2.0]non-2,4-diene-2-carboxylic acid :

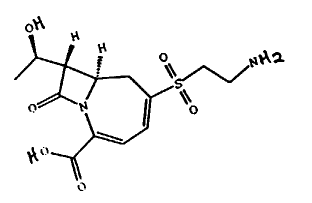

The title compound was prepared from 4-nitrobenzyl (7R,8S)-8-[(1R)-1-hydroxyethyl]-5-[2-(4-nitrobenzyloxycarbonyl)-aminoethylsulphonyl]-9-oxo-1-azabicyclo [5.2.0] non-2,4-diene-2-carboxylate by a similar procedure to that described in Example 1, method B.
1H NMR (400 MHz, D2O) δ:1.34 (d, 3H); 2.87 (ddd, 1H); 3.28 (dd, 1H); 3.41 (t, 2H); 3.49 (d, 1H); 3.63 (t, 2H); 3.82 (m, 1H); 4.38 (m, 1H); 5.78 (d, 1H); 7.18 (ddd, 1H).

The starting materials were prepared as follows :

4-Nitrobenzyl (7R, 8S)-8-[(1R)-1-(tert-butyldimethylsilyloxy)ethyl]-5-[2-(4-nitrobenzyloxycarbonyl)aminoethylsulphonyl]-9-oxo-azabicyclo[5.2.0]non-2,4-diene-2-carboxylate was prepared from 4-nitrobenzyl (7R,8S)-8-[(1R)-1-(tert-butyldimethylsilyloxy)ethyl]-5-[2-(4-nitrobenzyloxycarbonyl)aminoethylthio]-9-oxo-1i azabicyclo [5.2.0] non-2,4-diene-2-carboxylate by using a similar procedure to that described in Example 3.
1H NMR (400 MHz, CDCl3) δ:0.02 (s, 3H); 0.07 (s, 3H); 0.82 (s, 9H); 1.28 (d, 3H); 2.62 (m, 1H); 3.00 (m, 1H); 3.19-3.25 (m, 2H); 3.52 (d, 1H); 3.60-3.76 (m, 3H); 4.23 (m, 1H); 5.19 (s, 2H); 5.38 (s, 2H); 5.48 (m, 1H); 6.12 (d, 1H); 7.12 (dd, 1H); 7.49 (d, 2H); 7.59 (d, 2H); 8.22 (d, 2H); 8.23 (d, 2H).

4-Nitrobenzyl (7R, 8S)-8-[(1R)-1-hydroxyethyl]-5-[2-(4-nitrobenzyloxycarbonyl)aminoethylsulphonyl]-9-oxo-1-azabicyclo[5.2.0]non-2,4-diene-2-carboxylate was prepared from 4-nitrobenzyl (7R,8S)-8-[(1R)-1-(tert-butyldimethylsilyloxy)ethyl]-5-[2-(4-nitrobenzyloxycarbonyl)aminoethylsulphonyl]-9-oxo-1-azabicyclo [5.2.0] non-2,4-diene-2-carboxylate by a similar procedure to that described in Example 1, method B.
1H NMR (400 MHz, CDCl3+AcODd3) δ:1.38 (d, 3H); 2.70 (ddd, 1H); 3.08 (dd, 1H); 3.22-3.37 (m, 2H); 3.59-3.62 (m, 2H); 3.80 (dd, 1H); 4.35 (m, 1H); 5.18 (brs, 2H); 5.36 (d, 1H); 5.41 (d, 1H); 6.20 (d, 1H); 7.12 (dd, 1H); 7.49 (d, 2H); 7.59 (d, 2H); 8.20-8.25 (m, 4H).

## Claims

1. A compound of the formula (I): 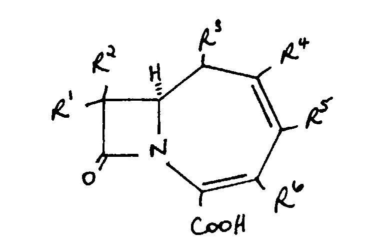
wherein R1 is hydrogen, optionally substituted: acylamino, C₁₋₆alkyl, C₂₋₆alkenyl, C₂₋₆alkynyl, C₁₋₆alkoxy, C₁₋₆alkanoyloxy, C₁₋₆alkylamino, di-(C₁₋₆alkyl)amino, C₁₋₆alkoxycarbonyl, aminocarbonyl, C₁₋₆alkylaminocarbonyl or di-(C₁₋₆alkyl)aminocarbonyl;
R² is hydrogen or C₁₋₆alkoxy;
or R¹ and R² together form optionally substituted C₁₋₆alkylene;
R³ and R⁵ are independently hydrogen or C₁₋₆alkyl; and
one of R⁴ and R⁶ is hydrogen or C₁₋₄alkyl and the other is selected from hydrogen, cyano, C₁₋₄alkylcarbonyl, C₁₋₄alkoxycarbonyl, benzoyl, C₁₋₄alkylsulphonyl, benzylsulphonyl, nitro, chloro, bromo, optionally substituted C₁₋₁₀alkyl, optionally substituted aryl, a group of the formula -SR⁷; wherein R⁷ is optionally substituted: C₁₋₁₀alkyl, C₂₋₆alkenyl, C₂₋₆alkynyl, C₃₋₁₀cycloalkyl, C₃₋₁₀cycloalkylC₁₋₃alkyl, aryl, arylC₁₋₃alkyl, heterocyclyl, heterocydylC₁₋₃alkyl, heteroaryl or heteroarylC₁₋₃alkyl;
or one of R⁴ and R⁶ is hydrogen or C₁₋₄alkyl and the other is of the formula -OR¹⁸, wherein R¹⁸ is hydrogen, optionally substituted:
C₁₋₆alkyl, aryl, C₁₋₆alkanoyl or arylcarbonyl;
or one of R⁴ and R⁶ is hydrogen or C₁₋₄alkyl and the other is of the formula -N(R¹⁹)R²⁰ wherein R¹⁹ and R²⁰ are independently hydrogen, optionally substituted C₁₋₆alkyl,
C₁₋₆alkanoyl, arylC₁₋₃alkanoyl, arylcarbonyl, heterocyclyl, heterocyclylC₁₋₃alkyl, heteroaryl, heteroarylC₁₋₃alkyl, arylC₁₋₃alkyl, cycloalkyl or cydoalkylC₁₋₃alkyl;
or one of R⁴ and R⁶ is hydrogen or C₁₋₄alkyl and the other is of the formula -CH₂R²¹ wherein R²¹ is aryl or of the formula -SR⁷, -OR¹⁸ or -N(R¹⁹)R²⁰ wherein R⁷ and R¹⁸⁻R²⁰ are as hereinabove defined;
or a pharmaceutically acceptable salt or in vivo hydrolysable ester thereof.

2. A compound according to claim 1 wherein R¹ is alkyl optionally substituted by halo, hydroxy, C₁₋₆alkylthio, C₁₋₆alkanoyl, C₁₋₆alkylsulphinyl, C₁₋₆akylsulphonyl, triazolyl, tetrazolyl or R¹ is acylamino.

3. A compound according to either claim 1 or claim 2 wherein R² is hydrogen.

4. A compound according to any one of claims 1 to 3 wherein R³ is hydrogen or methyl.

5. A compound according to any one of claims 1 to 4 wherein R⁵ is hydrogen.

6. A compound according to any one of claims 1 to 5 wherein one of R⁴ and R⁶ is hydrogen and the other is hydrogen, optionally substituted C₁₋₁₀alkyl, optionally substituted aryl, a group of the formula -S(O)ₙR⁷ wherein n is 0, 1 or 2 and R⁷ is optionally substituted:
C₁₋₆alkyl or heterocyclyl (wherein optional substituents on C₁₋₆alkyl are selected from amino, C₁₋₄alkylamino, di-(C₁₋₄alkyl)amino or of the formula: -N=C(R⁸)R⁹, -N(R¹⁰C(R¹¹)=N-R¹² and -C(N(R¹³)R¹⁴)=N-R¹⁵ wherein R⁸ is amino, C₁₋₄alkylamino or di-(C₁₋₄alkyl)amino and R⁹-R¹⁵ are independently hydrogen or C₁₋₄alkyl and optional substituents on heterocyclyl groups are selected from C₁₋₄alkyl, carboxy, C₁₋₄alkoxycarbonyl, -CON(R¹⁶)R¹⁷ wherein R¹⁶ is hydrogen or C₁₋₄alkyl and R¹⁷ is hydrogen, C₁₋₄alkyl, aryl, heteroaryl, heterocyclyl, cycloalkyl and cycloalkylC₁₋₃alkyl);
or one of R⁴ and R⁶ is of the formula -OR¹⁸ wherein R¹⁸ is as defined in claim 1;
or one of R⁴ and R⁶ is of the formula -N(R¹⁹)R²⁰ wherein R¹⁹ and R²⁰ are independently selected from hydrogen, C₁₋₄alkyl, C₁₋₄alkanoyl and aryl;
or of the formula -CH₂R²¹ wherein R²¹ is as defined in claim 1.

7. A compound according to claim 6 wherein one of R⁴ and R⁶ is C₁₋₄alkanoyloxy or of the formula -S(O)ₙR⁷ wherein n is 0 or 2 and R⁷ is C₁₋₆alkyl optionally substituted by amino or one of R⁴ and R⁶ is of the formula -SR⁷ wherein R⁷ is of the formula: 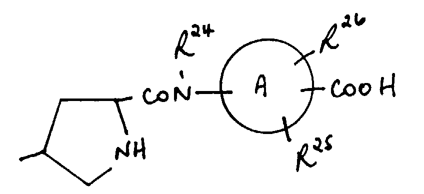
wherein R²⁴ is hydrogen or C₁₋₄alkyl and A is a phenyl or thienyl ring; and R²⁵ and R²⁶ are the same or different and are selected from hydrogen, halo, cyano, C₁₋₄alkyl, nitro, hydroxy, carboxy, C₁₋₄alkoxy, C₁₋₄alkoxycarbonyl, aminosulphonyl, C₁₋₄alkylaminosulphonyl, di-C₁₋₄₋ alkylaminosulphonyl, carbamoyl, C₁₋₄alkylcarbamoyl, di-C₁₋₄ alkylcarbamoyl, trifluoromethyl, sulphonic acid, amino, C₁₋₄alkylamino, di-C₁₋₄alkylamino,C₁₋₄alkanoylamino, C₁₋₄alkanoyl(N-C₁₋₄alkyl)amino, C₁₋₄alkanesulphonamido and C₁₋₄alkylS(O)ₙ₋ wherein n is zero, one or two.

8. A compound according to claim 1 which is:
(7R, 8S)-5-acetoxy-8-(1-hydroxyethyl)-9- oxo-1-azabicyclo[5.2.0]non-2,4-diene-2-carboxylic acid,
(7R, 8S)-8-((1R)-1-hydroxyethyl)-5-methylthio-9-oxo-1-azabicydo-[5.2.0]non-2,4-diene-2-carboxylic acid;
(7R, 8S)-8-((1R)-1-hydroxyethyl)-3-methanesulphonyl-9-oxo-1-azabicyclo[5.2.0]non-2,4-diene-2-carboxylic acid;
(7R, 8S)-5-(2-aminoethylthio)-8-((1R)-1-hydroxyethyl)-9-oxo-1-azabicyclo[5.2.0]-2,4-diene-2-carboxylic acid; or
(7R, 8S)-5-(2-aminoethylsulphonyl)-8-((1R)-1-hydroxyethyl)-9-oxo-1-azabicyclo[5.2.0]-2,4-diene-2-carboxylic acid; or a pharmaceutically acceptable salt thereof.

9. A pharmaceutical composition comprising a compound according to claim 1 and a pharmaceutically acceptable carrier.

10. A process for preparing a compound according to claim 1 which comprises deprotecting a compound of the formula (II): 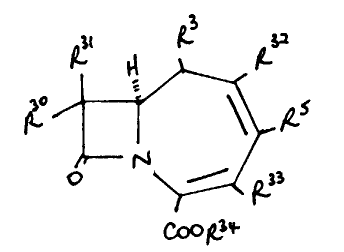
wherein R³ and R⁵ are as defined in claim 1; R³⁰ is R¹ or protected R¹; R³¹ is R² or R³⁰ and R³¹ together are optionally substituted C₁₋₆alkylene wherein any optional substituents are optionally protected; R³² is R⁴ or protected R⁴; R³³ is R⁶ or protected R⁶; and -COOR³⁴ is carboxy or protected carboxy; wherein R¹, R², R⁴ and R⁶ are as defined in claim 1 wherein at least one protecting group is present:
and thereinafter if necessary;
(i) forming a pharmaceutically acceptable salt,
(ii) esterifying to form an in vivo hydrolysable ester.

11. A process for preparing a compound according to claim 1 or a compound of the formula (II) as defined in claim 10 which comprises:
a) for compounds of the formula (I) wherein R⁴ is of the formula -SR⁷ or for compounds of the formula (II) wherein R³² is of the formula -SR^{7'}wherein R^{7'} is R⁷ or protected R⁷, reacting a compound of the formula HSR^{7'}(compound B) with a compound of the formula (III) 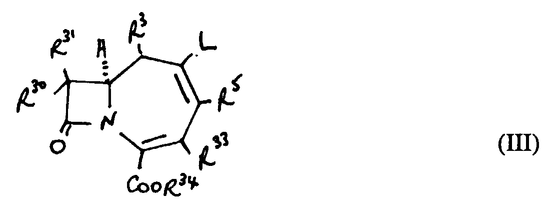
wherein R³, R⁵, R³⁰, R³¹, R³³ and R³⁴ are as hereinabove defined and L is a leaving group;
b) for compounds of the formula (I) wherein R⁴ is of the formula -OR¹⁸ or for compounds of the formula (II) wherein R³² is of the formula -OR¹⁸' wherein R¹⁸' is R¹⁸ or protected R¹⁸; reacting a compound of the formula HOR^{18'} (compound D) with a compound of the formula (III) wherein L, R³, R⁵, R³⁰, R³¹, R³³ and R³⁴ are as hereinabove defined;
c) for compounds of the formula (I), wherein R⁴ is of the formula -N(R¹⁹)R²⁰ or for compounds of the formula (II) wherein R³² is of the formula -N(R^{19'})R^{20'} wherein -N(R^{19'})R^{20'} is -N(R¹⁹)R²⁰ or protected -N(R¹⁹)R²⁰:
(i) reacting a compound of the formula HN(R^{19'})R^{20'} (compound E) with a compound of the formula (III)
wherein L, R³, R⁵, R³⁰, R³¹, R³³ and R³⁴ are as hereinabove defined; or
ii) converting a compound of the formula (IIIA): 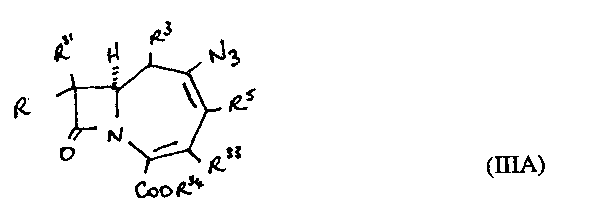 wherein R³, R⁵, R³⁰, R³¹, R³³ and R³⁴ are as hereinbefore defined to a compound of the formula (I) or (II);
d) for compounds of the formula (I), wherein R⁴ is optionally substituted C₁₋₁₀alkyl, optionally substituted aryl or of the formula -CH₂R²¹ or compounds of the formula (II) wherein R³² is optionally substituted C₁₋₁₀alkyl, optionally substituted aryl, protected substituted C₁₋₁₀alkyl, protected aryl or is of the formula -CH₂R^{21'} wherein R^{21'} is R²¹ or protected R²¹; reacting a compound
of the formula R³⁵R³⁶R³⁷SnR³⁸ (compound F) with a compound of the formula (III) wherein L, R³, R⁵, R³⁰, R³¹, R³³ and R³⁴ are as hereinabove defined and R³⁵⁻R³⁷ are C₁_₆alkyl and R³⁸ is optionally substituted C₁₋₁₀alkyl, aryl or of the formula -CH₂R^{21'};
e) cyclising a compound for the formula (IV): 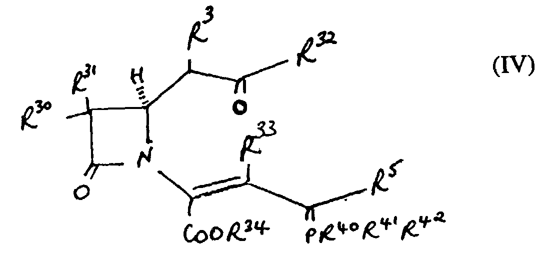 wherein R³,R⁵,R³⁰-R³⁴ are as defined in claim 10 and R⁴⁰-R⁴² are independently selected from C₁₋₆alkoxy, phenyl and phenoxy, wherein any phenyl group is optionally substituted with C₁₋₃alkyl or C₁₋₃alkoxy;
and wherein any functional group is optionally protected and thereinafter if necessary:
(i) removing any protecting groups;
(ii) forming a pharmaceutically acceptable salt;
(iii) esterifying to form an in vivo hydrolysable ester.

12. A compound of the formula (II) as defined in 10, or a compound of the formula (III), (IIIA) or (IV) as defined in claim 11.
